(19) Europäisches Patentamt
European Patent Office
Office européen des brevets

(11) Publication number: **0 310 647 B1**

# (12) EUROPEAN PATENT SPECIFICATION

(45) Date of publication of patent specification: **09.12.92**

(51) Int. Cl.5: **C07F 5/02**

(21) Application number: **88903334.6**

(22) Date of filing: **08.04.88**

(86) International application number:
**PCT/HU88/00018**

(87) International publication number:
**WO 88/07998 (20.10.88 88/23)**

## (54) OUINOLINE CARBOXYLIC ACID BORIC ACID ANHYDRIDES AND PROCESS FOR THE PREPARATION THEREOF.

(30) Priority: **08.04.87 HU 150487**
**24.06.87 HU 285787**
**10.07.87 HU 314787**

(43) Date of publication of application:
**12.04.89 Bulletin 89/15**

(45) Publication of the grant of the patent:
**09.12.92 Bulletin 92/50**

(84) Designated Contracting States:
**AT BE CH DE FR GB IT LI LU NL SE**

(56) References cited:
**WO-A-87/03587**
**JP-A-59 122 470**

(73) Proprietor: **CHINOIN Gyogyszer és Vegyészeti Termékek Gyára RT.**
**To utca 1-5**
**H-1045 Budapest IV(HU)**

(72) Inventor: **HERMECZ, István**
**Molnár u. 53**
**H-1056 Budapest(HU)**
Inventor: **KERESZTURI, Géza**
**Széna tér 1/b**
**H-1015 Budapest(HU)**
Inventor: **VASVARI, Lelle**
**Goldmark K.u. 33**
**H-1122 Budapest(HU)**
Inventor: **HORVATH, Agnes**
**Budenz u. 30/a**
**H-1021 Budapest(HU)**
Inventor: **BALOGH, Mária**
**Barátság u. 20**
**H-2120 Dunakeszi(HU)**
Inventor: **RITLI, Péter**
**O u. 43**
**H-1066 Budapest(HU)**
Inventor: **SIPOS, Judit**
**Sáfrány u. 10**
**H-1116 Budapest(HU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **PAJOR, Aniko**
**Ferenc krt. 36**
**H-1092 Budapest(HU)**
Inventor: **MARMAROSI, Katalin**
**Ybl M. st. 19**
**H-2051 Biatorbágy(HU)**

(74) Representative: **Skailes, Humphrey John et al**
**Frank B. Dehn & Co. Imperial House 15-19**
**Kingsway**
**London WC2B 6UZ(GB)**

## Description

This invention relates to new (1-cyclopropyl-6-fluoro-7-chloro-8-optionally fluoro-substituted-4-oxo-1,4-dihydro-quinoline-3-carboxylic-acid-boric acid)-anhydride derivatives of the general Formula I

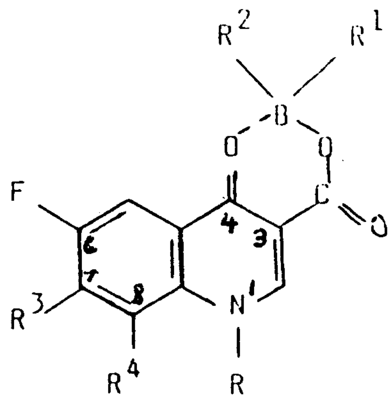

(I)

and to the preparation thereof. In the general Formula I

| | |
|---|---|
| R | is cyclopropyl, |
| $R^1$ and $R^2$ | are the same or different and are aliphatic acyloxy groups containing 2 to 6 carbon atoms, optionally substituted by halogen, or aromatic acyloxy groups containing 7 to 11 carbon atoms, |
| $R^3$ | is chlorine and |
| $R^4$ | is hydrogen or fluorine. |

Ethyl-(1-cyclopropyl-6-fluoro-7-chloro-4-oxo-1,4-dihydro-quinoline-3-carboxylate) is an intermediate for the preparation of 1-cyclopropyl-6-fluoro-1,4-dihydro-4-oxo-7-(1-piperazinyl)-quinoline-3-carboxylic acid which has antibacterial activity (Eur.J.Clin.Microbiol. 1983., 2, 111). The latter compound can be obtained from ethyl-(1-cyclopropyl-6-fluoro-7-chloro-4-oxo-1,4-dihydro-quinoline-3-carboxylate) in two steps. After hydrolysing the ester, the 1-cyclopropyl-6-fluoro-7-chloro-1,4-dihydro-4-oxo-quinoline-3-carboxylic acid thus obtained is reacted with piperazine in the presence of a solvent at a temperature of 135-140 °C for two hours (German Off. No. 30 33 157 and No. 31 42 854).

JP-A-59122470 describes the preparation of 1-alkyl compounds of this type from $BF_2$-complexes of corresponding 7-halo compounds. We have found that 1-cyclopropyl-6-fluoro-1,4-dihydro-4-oxo-7-/optionally substituted piperazino/-quinoline-3-carboxylic acid can be prepared in a simple manner by using the new compounds of general Formula I as starting material.

The compounds of the general Formula I can be obtained by reacting a compound of the general Formula

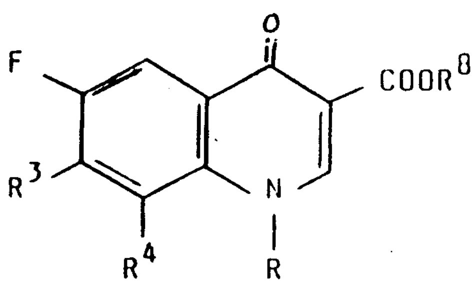

(II)

(wherein R, $R^3$ and $R^4$ are as stated above and $R^8$ stands for hydrogen or alkyl containing 1 to 4 carbon with a triacyloxy borate derivative of the general Formula V

$B(OCOR^9)_3$ (V)

(wherein $R^9$ stands for alkyl containing 1 to 5 carbon atoms, optionally substituted by halogen, or aryl

containing 6 to 10 carbon atoms).

The triacyloxy borate derivatives of the general Formula V can be used in a molar ratio of 50:1, preferably 5:1, relative to the compounds of the general Formula II. However, if desired, other molar ratio may also be used.

The reaction may also be carried out in the presence of a solvent such as water, ketones (e.g. acetone, methyl ethyl ketone), hydrocarbons (e.g. hexane, benzene, toluene), ethers (e.g. diethyl ether, dioxane, tetrahydrofuran) or organic acids (e.g. acetic acid, propionic acid, trifluoroacetic acid).

If desired the reagents can be reacted at room temperature. If the reaction is carried out at higher temperature, the reaction time can be shortened. One may preferably carry out the reaction at a temperature ranging from 20 to 150 °C, but the reaction temperature depends on the solvent applied.

The compounds of the general Formula I precipitate from the reaction mixture spontaneously or on cooling and thus can be separated e.g. by filtration.

Further details of the present invention are to be found in the following Example

Example 1

4.6 g of acetic acid are added to the mixture of 0.93 g of boric acid and 1.5 mg of zinc chloride and the suspension is heated slowly under stirring. A clear solution is obtained at 80 °C. A solution of 3.1 g of ethyl-(1-cyclopropyl-6-fluoro-7-chloro-1,4-dihydro-4-oxo-quinoline-3-carboxylate) in 5 ml of hot 96 w/v% acetic acid is added dropwise to the clear solution at 100 °C. The reaction mixture is stirred at 110 °C for two hours, then cooled to 10 °C and diluted with 10 ml of water. The suspension thus obtained is stirred at 10 °C for two hours. The precipitated cream-coloured crystals are filtered, washed with water and ethanol and dried. Thus 4.02 g of (1-cyclopropyl-6-fluoro-7-chloro-1,4-dihydro-4-oxo-quinoline-3-carboxylate-$0^3$,$0^4$)-bis-(acetato-0)-boron are obtained. The product decomposes at 254-256 °C.

| Analysis for the Formula $C_{17}H_{14}O_7NBClF$: | | | |
|---|---|---|---|
| Calculated: | C = 49.86% | H = 3.45% | N = 3.42% |
| Found: | C = 50.03 | H = 3.41% | N = 3.50%. |

**Claims**

**1.** Process for the preparation of compounds of the general Formula I

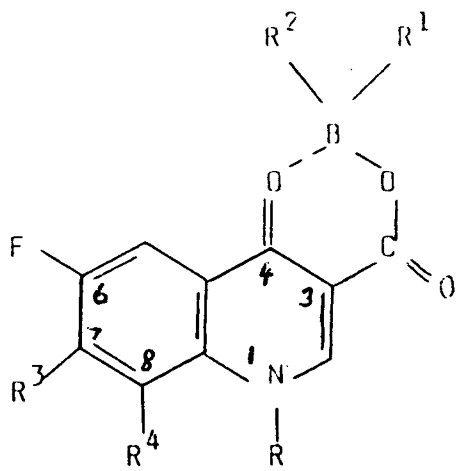

(I)

(wherein

| | |
|---|---|
| R | is cyclopropyl, |
| $R^1$ and $R^2$ | are aliphatic acyloxy groups containing 2 to 6 carbon atoms, optionally substituted by halogen, or aromatic acyloxy groups containing 7 to 11 carbon atoms, |
| $R^3$ | is chlorine and |
| $R^4$ | is hydrogen of fluorine), which comprises reacting a compound of the general Formula |

II

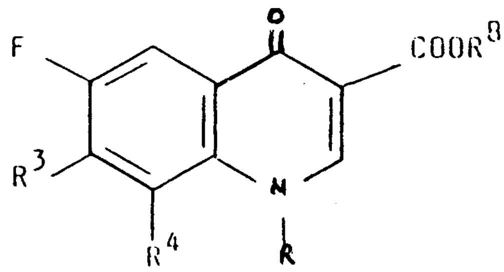

(II)

(wherein R, $R^3$ and $R^4$ are as stated above and $R^8$ stands for hydrogen of alkyl containing 1 to 4 carbon atoms) with a triacyloxy borate derivative of the general Formula V

$B(OCOR^9)_3$ (V)

(wherein $R^9$ stands for alkyl containing 1 to 5 carbon atoms, optionally substituted by halogen, or aryl containing 6 to 10 carbon atoms).

**2.** Process according to Claim 1 which comprises reacting a compound of the general Formula II with a compound of the general Formula V in the presence of a solvent.

**3.** Compounds of the general Formula I

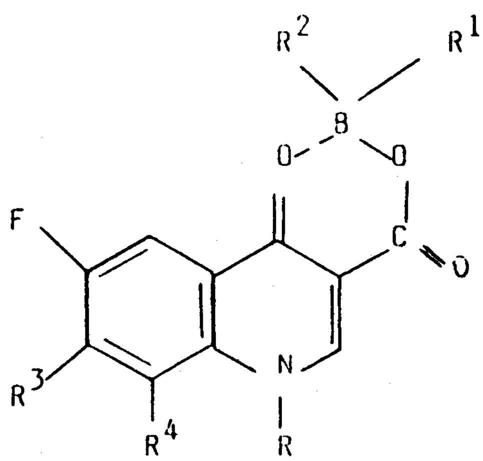

(I)

wherein

| | |
|---|---|
| R | is cyclopropyl, |
| $R^1$ and $R^2$ | are aliphatic acyloxy groups containing 2 to 6 carbon atoms optionally substituted by halogen, or aromatic acyloxy groups containing 7 to 11 carbon atoms, |
| $R^3$ | is chlorine and |
| $R^4$ | is hydrogen or fluorine. |

**Patentansprüche**

**1.** Verfahren zur Herstellung von Verbindungen der allgemeinen Formel I

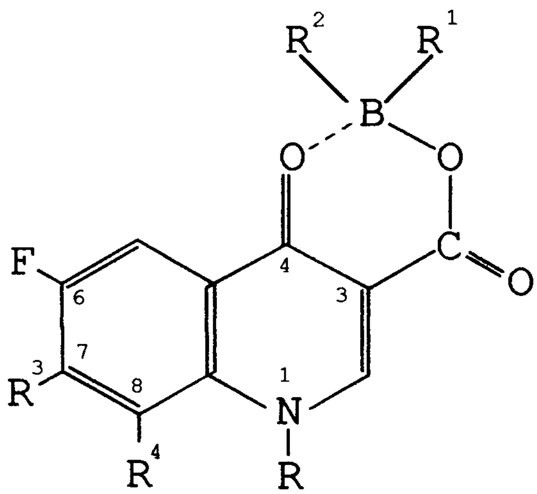

(I)

(worin

| | |
|---|---|
| R | Cyclopropyl, |
| $R^1$ und $R^2$ | aliphatische $C_2$-$C_6$-Acyloxigruppen, die gegebenenfalls durch Halogen substituiert sein können, oder aromatische $C_7$-$C_{11}$-Acyloxigruppen, |
| $R^3$ | Chlor und |
| $R^4$ | Wasserstoff oder Fluor bedeuten) |

dadurch gekennzeichnet, daß man eine Verbindung der allgemeinen Formel II

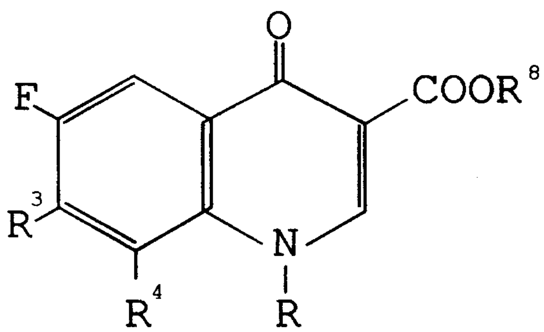

(II)

(worin

| | |
|---|---|
| R, $R^3$ und $R^4$ | die oben angegebene Bedeutung haben, und |
| $R^8$ | Wasserstoff oder $C_1$-$C_4$-Alkyl bedeuten) mit einem Triacyloxi-borat-derivat der allgemeinen Formel V |

$B(OCOR^9)_3$ (V)

(worin

| | |
|---|---|
| $R^9$ | $C_1$-$C_5$-Alkyl, das gegebenenfalls durch Halogen substituiert sein kann,oder $C_6$-$C_{10}$-Aryl bedeutet) |

umsetzt.

2. Verfahren nach Anspruch 1, dadurch gekennzeichnet, daß man die Umsetzung der Verbindung der allgemeinen Formel II mit einer Verbindung der allgemeinen Formel V in Gegenwart eines Lösungsmittels durchführt.

3. Verbindungen der allgemeinen Formel I

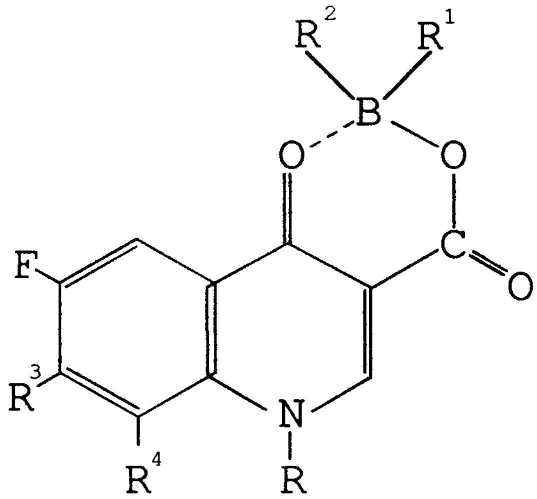

(I)

(worin

| | |
|---|---|
| R | Cyclopropyl, |
| $R^1$ und $R^2$ | aliphatische $C_2$-$C_6$-Acyloxigruppen, die gegebenenfalls durch Halogen substituiert sein können, oder aromatische $C_7$-$C_{11}$-Acyloxigruppen, |
| $R^3$ | Chlor und |
| $R^4$ | Wasserstoff oder Fluor bedeuten). |

## Revendications

**1.** Procédé pour la préparation de composés de Formule générale I :

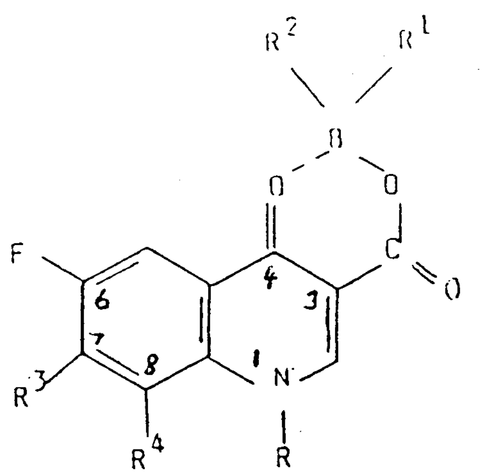

(I)

dans laquelle :

| | |
|---|---|
| R | est le cyclopropyle, |
| $R^1$ et $R^2$ | sont des groupements aliphatiques acyloxy contenant de 2 à 6 atomes de carbone, éventuellement substitué par un halogène, ou des groupements aromatiques acyloxy contenant de 7 à 11 atomes de carbone, |
| $R^3$ | est un atome de chlore et |
| $R^4$ | est un atome d'hydrogène ou de fluor, |

qui comprend de faire réagir un composé de Formule générale II

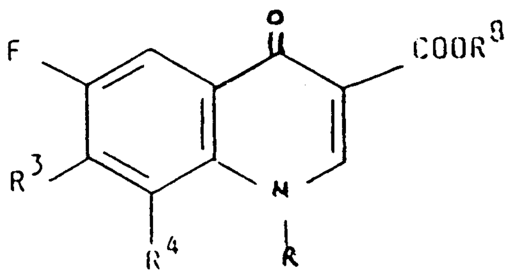

(II)

dans laquelle R, $R^3$ et $R^4$ sont comme définis ci-dessus et $R^8$ correspond à un atome d'hydrogène ou un groupement alkyle contenant de 1 à 4 atomes de carbone
avec un dérivé de triacyloxy borate de Formule générale V

$B(OCOR^9)_3$ (V)

dans laquelle $R^9$ correspond à un groupement alkyle contenant de 1 à 5 atomes de carbone, éventuellement substitué par un halogène, ou à un groupement aryle contenant de 6 à 10 atomes de carbone.

2. Procédé selon la revendication 1 qui comprend de faire réagir un composé de la Formule générale II avec un composé de la Formule générale V en présence d'un solvant.

3. Composés de Formule générale I

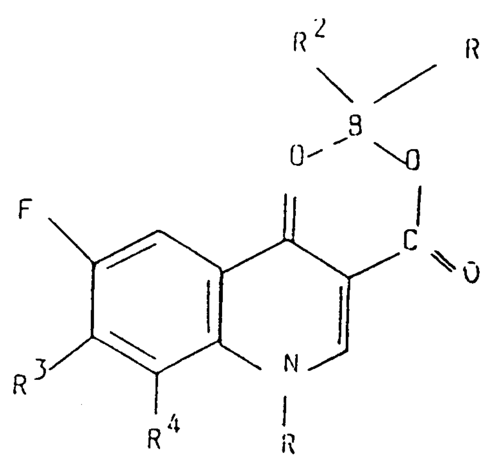

(I)

dans laquelle :

| | |
|---|---|
| R | est le cyclopropyle, |
| $R^1$ et $R^2$ | sont des groupements aliphatiques acyloxy contenant de 2 à 6 atomes de carbone, éventuellement substitué par un halogène, ou des groupements aromatiques acyloxy contenant de 7 à 11 atomes de carbone, |
| $R^3$ | est un atome de chlore et |
| $R^4$ | est un atome d'hydrogène ou de fluor. |